(19) Europäisches Patentamt
European Patent Office
Office européen des brevets

(11) EP 3 626 174 A1

(12) EUROPEAN PATENT APPLICATION

(43) Date of publication:
25.03.2020 Bulletin 2020/13

(51) Int Cl.:
A61B 6/00 (2006.01)
G02F 1/11 (2006.01)
G02F 1/33 (2006.01)
G02B 27/50 (2006.01)
G01N 23/041 (2018.01)
G21K 1/04 (2006.01)
G01V 5/00 (2006.01)
G02B 27/52 (2006.01)
G01N 23/20 (2018.01)
G02F 1/29 (2006.01)
G02B 5/18 (2006.01)
G21K 1/06 (2006.01)
G02F 1/165 (2019.01)
G03F 7/20 (2006.01)
G02B 21/14 (2006.01)

(21) Application number: 18195665.7

(22) Date of filing: 20.09.2018

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(71) Applicant: Koninklijke Philips N.V.
5656 AG Eindhoven (NL)

(72) Inventors:
• STEADMAN BOOKER, Roger
5656 AE Eindhoven (NL)
• ROESSL, Ewald
5656 AE Eindhoven (NL)
• VERSTEEG, Dennis John
5656 AE Eindhoven (NL)

(74) Representative: de Haan, Poul Erik et al
Philips International B.V.
Philips Intellectual Property & Standards
High Tech Campus 5
5656 AE Eindhoven (NL)

(54) **SWITCHABLE GRATING**

(57) A switchable grating for phase contrast imaging comprising a reservoir with a medium and x-ray absorbing particles acoustically connected to a first ultrasound generator and a second ultrasound generator arranged along a side of the reservoir orthogonal to the first side. The ultrasound generators are each, individually or together, configured to generate a soundwave with a frequency and phase such that a standing wave is formed within the medium causing the x-ray absorbing particles to organize along pressure nodes of the standing waves.

Fig. 3

## Description

FIELD OF THE INVENTION

The present invention generally relates to a switchable grating for a phase contrast imaging and/or dark-field imaging interferometer, a system for differential phase contrast imaging and/or dark-field imaging and a for differential phase contrast imaging and/or dark-field imaging.

BACKGROUND OF THE INVENTION

Phase contrast imaging, such as dark-field x-ray imaging (DAX) and differential phase contrast imaging (DCPI), provide high sensitivity to phase-gradients and scattering structures in an object and are a promising addition to diagnostic or analytical (x-ray) imaging, for instance for medical diagnoses, material analysis and security scanning.

Phase contrast imaging (this term is used throughout this document to cover both DAX and DCPI) is an imaging technique that only recently found practical use in x-ray imaging. Phase contrast imaging is already long known for visual optics, but for x-ray imaging it was restricted to high brilliance synchrotron x-ray sources that are not suitable for practical (e.g. medical) imaging due to their size and very limited energy band width and angular divergence. However, a grating-based solution was developed to generate dark field x-ray images using x-ray tubes commonly used in medical imaging and security scanning [Pfeiffer et.al., "Hard-X-ray dark-field imaging using a grating interferometer", Nature Materials, Vol. 7, February 2008, page 134-137].

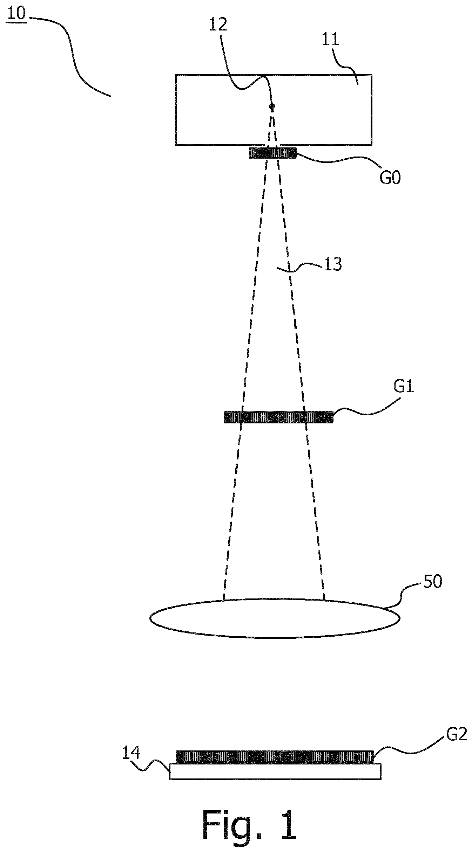

Phase contrast imaging may be performed using a phase contrast set-up 10 as is schematically shown in fig. 1. An X-ray beam 13 is emitted from an x-ray focal spot 12 of an x-ray source 11. A first grating structure G0, commonly named the source grating, is placed close to the x-ray focal spot 12. Because of the source grating G0 the radiation beam 13 is in effect divided into an array of line sources splitting up the beam into separate parallel beams, which then pass through a second grating structure G1, commonly named the phase grating, which may be placed in front (e.g. for computed tomography (CT) imaging) or behind (e.g. for standard x-ray imaging) an object 50 to be imaged. The x-ray beam 13 passes a third grating structure G2, commonly named the analyzer grating or the amplitude grating, before it is detected by a detector 14, in which imaging information is generated and transmitted to processing equipment (not shown). Both the phase grating G1 and the analyzer grating G2 contribute to image contrast, wherein the phase grating G1 causes periodic spatial phase modulations in the x-ray beam 13. By propagation of the partially coherent x-rays, the phase modulation is transformed into an intensity modulation which has a typical period in the range of 5 - 50 $\mu$m (Talbot effect). The analyzer grating G2 then transforms the highfrequency intensity modulations into a low-frequency intensity modulation. When the phase grating G1 or the analyzer grating G2 is moved transversely with respect to the radiation beam 13, the x-ray intensity detected oscillates in each pixel of the detector 14, wherein the magnitude of the oscillations is determined by the object 50. These local intensity changes may then be used to determine dark field and phase contrast image data, which are obtained simultaneously.

The obtained dark field image data represents scatter information of the x-ray beam 13 through the object 50. This scatter data is obtained simultaneously with x-ray transmission image data, which provides attenuation measurement data, particularly of a difference between high and low absorption areas, and with phase contrast image data, which provides increased soft-tissue contrast, which makes it particularly suitable for imaging 'soft' materials with a lot of surface area transitions and/or micro-structures (e.g. lungs, fibrous materials and the like).

The obtained phase contrast image represents refractive index information of the x-ray beam 13 through the object 50. This may be advantageously used, on its own or in combination with the also simultaneously obtained transmission image, to enhance image contrast by using detailed differing refractive index changes within structures that are otherwise uniform.

The described phase contrast set-up 10 is very suitable for this purpose, but the claimed invention is suitable for any grating-based phase contrast set-up suitable for medical or analytical imaging.

A CT or other x-ray scanner capable of phase contrast imaging normally requires practically integrating G0, G1 and G2 gratings between the source 11 and the detector 14. These gratings attenuate radiation and need to be removed from the path of the radiation beam when the imaging device is used for conventional x-ray imaging. This is in order to ensure proper dose usage, as G2 typically discards e.g. 50% of the dose that an object (e.g. a human patient in a diagnostic scan) has already been exposed to and the attenuation by G0 and G1 requires the source to emit at a much higher flux to obtain transmission images with a quality comparable when no gratings would be present. While G0 and G1 can be placed on, for instance, A-plane trays and moved out of the beam path, G2 is much more sensitive to mechanical precision. Failing to place the G2 in the same position within micrometer tolerance will entail having to re-calibrate the system.

A potential solution may be to use a grating that may be switched on and off without physically having to remove or displace the grating hardware. An example of this may be found in US2007/0183584 A1, in which a switchable grating is formed by generating standing waves in a medium that organizes itself into periodical structures. The medium may be a suspension of x-ray absorbing particles and the standing waves may be induced using an ultrasound generator.

While this system is adaptable for use in CT or other x-ray phase contrast imaging, it only allows for unidirectional gratings, with which it is only possible to resolve phase gradients and scattering structures in one distinct direction. To overcome this in known systems the analyzer grating G2 needs to be rotated 90 degrees (accompanied by also a 90 degrees rotation of either the source grating G0 or the phase grating G1). This would require mechanical rotation of the gratings, which is relatively easy for the source grating G0 and the phase grating G1, but due to the highly demanding required precision is problematic for the analyzer grating G2 for the same reasons as described previously for removing the grating, particularly since the known switchable grating is a more bulky system comprising a container with a (liquid) medium connected to an ultrasound generator.

SUMMARY OF THE INVENTION

The presently claimed invention provides a solution to the above-mentioned problems and more.

Embodiments according to the present invention are directed towards a switchable grating for a differential phase contrast imaging and/or dark-field imaging interferometer comprising a reservoir containing a substantially x-ray transparent medium comprising particles that substantially attenuate x-ray radiation; a first ultrasound generator arranged along a first side of the reservoir configured to generate a first soundwave with a frequency and phase such that a first standing wave is formed within the medium causing the x-ray absorbing particles to organize along pressure nodes of the first standing wave; and a second ultrasound generator arranged along a second side of the reservoir orthogonal to the first side configured to generate a second soundwave with a frequency and phase such that a second standing wave is formed within the medium causing the x-ray absorbing particles to organize along pressure nodes of the second standing wave.

The second standing wave is generated in a direction orthogonal to the first standing wave thereby forming a grating structure that is also orthogonal with respect to one that be generated with the first standing wave. As such the grating may be generated in positions orthogonal with respect to each other. This allows for phase contrast imaging of an object in two directions, thereby allowing for detecting (local) anisotropic differences in structures in the object.

In an embodiment the first ultrasound generator and second ultrasound generator are configured to operate separately, alternatingly and/or simultaneously. This allows a high degree of freedom in imaging an object with wall-shaped gratings in two orthogonal directions or to generate pillars when both generators are switched on. When operated alternatingly it may be possible to obtain optimally registered images of both anisotropic information of structures in the object.

In an embodiment the first ultrasound generator and second ultrasound generator are configured to independently generate a soundwave at a selected frequency. In other words: the ultrasound generators may each be operated at the same or at different frequencies. By changing the frequency it is possible to influence the position and spacing of the formed grating structures.

In an embodiment the first ultrasound generator and/or the second ultrasound generator comprise at least two ultrasound transducers spaced next to each other, each of said transducers configured to independently generate a soundwave at a selected frequency. This allows for an even further increased degree of freedom in generating grating structures, particularly when used concurrently, forming differently patterned grating structures in different sections of the reservoir.

In an embodiment the switchable grating further comprises a third ultrasound generator arranged along a third side of the reservoir opposite to the first side configured to generate the first soundwave with a frequency and phase such that the first standing wave is formed within the medium causing the particles to organize along pressure nodes of the first standing wave; and/or a fourth ultrasound generator arranged along a fourth side of the reservoir opposite to the second side configured to generate the second soundwave with a frequency and phase such that the second standing wave is formed within the medium causing the particles to organize along pressure nodes of the second standing wave. Such additional ultrasound generators placed opposite the first and/or second ultrasound generators may assist in forming standing waves over the length and/or width of the reservoir in case only on ultrasound generator in one direction is not sufficient to maintain a standing wave over the length or width of the reservoir.

Further embodiments of the present invention is directed towards a switchable grating for a differential phase contrast imaging and/or dark-field imaging interferometer comprising a reservoir containing a substantially x-ray transparent medium comprising particles that substantially attenuate x-ray radiation; a first ultrasound generator arranged along a first side of the reservoir configured to generate a first soundwave; and means for flushing the reservoir to remove the particles, and optionally the medium, outside a field-of-view of an x-ray beam as used in an imaging procedure. As such the switchable grating does not need to be removed when the imager is used in normal transmission mode instead of a phase contrast imaging procedure. The known switchable grating absorbs radiation when not switched on since the particles are still present in the medium. By flushing them from the field-of-view of the x-ray beam there is no, or least almost no, attenuation by the switchable grating itself, such that it does not need to be removed from the path of the radiation beam and no repositioning or recalibration is necessary when the imager is used in phase contrast mode again.

In an embodiment the means for flushing the reservoir is arranged to flush the particles, and optionally the medium, to a side of the reservoir or to a container removed from the reservoir. As such the particles, and optionally the medium, are removed from the field-of-view of the x-ray beam used in the procedure.

In an embodiment the means for flushing the reservoir is configured to flush the particles, and optionally the medium, by generating a propagating soundwave from the first ultrasound generator such that the propagating soundwave carries substantially all particles outside a field-of-view of an x-ray beam during an imaging procedure. As such no additional hardware is necessary to flush the reservoir since the already present ultrasound generator is used to flush the particle.

Further embodiments of the present invention is directed towards a system for differential phase contrast imaging and/or dark-field imaging comprising a coherent polychromatic or an incoherent polychromatic x-ray source provided with a source grating in transmission geometry for making the x-ray beam coherent; an interferometer comprising at least a phase grating in transmission geometry for generating a pattern of interference fringes at a certain location downstream the optical axis; an x-ray detector having sufficient spatial resolution for resolving a fringe pattern generated by phase grating or a conventional X-ray detector provided with an analyzer grating in transmission geometry for resolving the interference fringes generated by the phase grating, wherein the interferometer further comprises, when present, the source grating, and/or the analyzer grating, and the interferometer is installed between the x-ray source and x-ray detector; characterized in that at least one of the phase grating or, when present, the source grating or, when present, analyzer grating is a switchable grating according to any of the previous claims. As such a full phase contrast imaging system is obtained with a switchable grating as presently claimed.

In an embodiment the analyzer grating is the switchable grating. While the switchable gating as claimed would be suitable for the source grating and phase grating as well, the most benefit is obtained for the analyzer grating, since this grating requires extremely precise positioning and calibration and not having to remove the grating for non-phase contrast procedures or repositioning of the gratings saves a lot of valuable time and effort and reduces the chance of errors.

In an embodiment at the phase grating and, when present, the source grating are rotatable by 90 degrees or are switchable gratings as claimed. When the analyzer grating is switched to an orthogonal direction, then also the other gratings need to be rotate by 90 degrees to be able to properly perform the phase contrast procedure. By also using a switchable grating this also increases speed of changing the orientation much higher and less cumbersome and less prone to errors.

Further embodiments of the presently claimed invention are directed towards a method for differential phase contrast imaging and/or dark-field imaging of an object comprising the steps of placing the object in an examination area of a system for differential phase contrast imaging and/or dark-field imaging as claimed; generating a first soundwave in a first direction with a frequency and phase such that a first standing wave is formed within the medium causing the particles to organize along pressure nodes of the first standing wave, thereby forming the analyzer grating; and/or generating a second soundwave in a second direction, orthogonal to the first direction, with a frequency and phase such that a second standing wave is formed within the medium causing the particles to organize along pressure nodes of the first standing wave, thereby forming the analyzer grating. This allows for a much faster, more efficient and precise method to select and prepare a phase contrast imaging procedure in different directions.

In an embodiment the first soundwave is switched off followed by generation of the second soundwave, preferably after the phase grating and, when present, the source grating are rotated by 90 degrees from a first position to a second position or are switchable gratings as claimed that are switched between a sound wave in the first direction to a second soundwave in the second direction, corresponding to the respective first direction and second direction of the switchable analyzer grating. This allows for a single phase contrast imaging procedure in respective different directions.

In an embodiment the first soundwave and the second soundwave are generated simultaneously. This allows for the formation of pillar-like grating structures instead of wall-like or slab-like grating structures.

In a further embodiment a grating for differential phase contrast imaging and/or dark-field imaging is manufactured comprising the method for differential phase contrast imaging and/or dark-field imaging as claimed, wherein the particles are curable particles that are cured after the particles have organized themselves along the pressure nodes of the standing wave(s) in a curing step, preferably a thermal curing step or a radiation curing step. As such permanent and highly accurate grating structures, such as wall, slabs or pillars, may be formed that may be used as (non-switchable) gratings in phase contrast imaging or other optical procedures. Since production of gratings with a high aspect ratio and high requirements regarding precision is difficult and commonly used materials (such as pure gold) are very expensive, this method may provide a cheaper, faster alterative to produce gratings of similar quality.

Still further aspects and embodiments of the present invention will be appreciated by those of ordinary skill in the art upon reading and understanding the following detailed description. Numerous additional advantages and benefits will become apparent to those of ordinary skill in the art upon reading the following detailed description of preferred embodiments.

BRIEF DESCRIPTION OF THE DRAWINGS

The present invention is illustrated by drawings of which

Fig. 1 shows a schematic depiction of a phase contrast imaging system.
Fig. 2a-c shows a schematic depiction of a known switchable grating.
Fig. 3a-f shows a schematic depiction of embodiments of a switchable grating in use as claimed.
Fig. 4a-c shows a schematic depiction of further embodiments of a switchable grating in use as claimed.
Fig. 5 shows a schematic depiction of embodiments of flushing a switchable grating as claimed.

The drawings are only for the purpose of illustrating preferred embodiments and are not to be construed as limiting the invention. To better visualize certain features may be omitted or dimensions may be not be according to scale.

DETAILED DESRIPTION OF EMBODIMENTS

The presently claimed invention is described with a focus on the analyzer grating G2 as being the switchable grating 20. However, the switchable grating 20 as claimed may also be used for the source grating G0 or the phase grating G1 or for two or all three of the gratings G0, G1, G2.

The presently claimed invention is described with a focus on the phase contrast arrangement as shown in Fig. 1 and as described earlier, but could also be used for alternate arrangements, such as a phase contrast set-up with less phase gratings or where the phase grating G1 is placed behind the object 50.

The detector 14 employed in the phase contrast arrangement according to the presently claimed invention may have a pitch sufficiently small, hence a spatial resolution sufficiently large, for detecting, i.e. adequately resolving the interference pattern generated by the phase grating G1. For that purpose such detection unit may comprise a high resolution x-ray detector 14 known per se having a resolution of 50 micrometers or more, or an x-ray detector 14 of the type as described in US 2014/0177795 A1 which is incorporated herein by reference. Alternatively, the detection unit may comprise an x-ray detector 14 having less high resolution, however, in conjunction with an analyzer grating G2, i.e. an absorption grating arranged in the optical path between the phase grating G1 and said x-ray detector 14.

In case no analyzer grating G2 is used, then also G0 may optionally be removed.

The interferometer employed in the phase contrast arrangement according to the present invention may be implemented by various geometries. The interferometer may comprise (i) (optionally, depending on the x-ray source) a source grating G0 with pitch po, (ii) a phase grating G1 with pitch $p_1$ and installed between the source grating G0 and the detector 14, and (iii) (optionally, depending on the implementation of the detection unit 14) an analyzer grating G2 with pitch $p_2$ and installed between the phase grating G1 and the detector. Introducing s as the distance between the source grating G0 and the analyzer grating G2 (if any), 1 as the distance between the source grating G0 and the phase grating G1 and d as the distance between the phase grating G1 and the analyzer grating G2 (if any), the various geometries are defined on the basis of said quantities. As a first option, the interferometer may be implemented in the so-called "conventional geometry" in which 1 > d and $p_0 > p_1 > p_2$. In the conventional geometry, the object to be imaged is typically arranged between the source grating G0 and the analyzer grating G1. As a second option, the interferometer may be implemented in the so-called "inverse geometry" in which 1 < d and $p_0 < p_1 < p_2$. In the inverse geometry, the object to be imaged is typically arranged between the phase grating G1 and the x-ray detection unit 14 (i.e. between the phase grating G1 and, if present, the analyzer grating G2). As a third option, the interferometer may be implemented in the so-called "symmetric geometry" in which d = 1 and $p_0 = p1 = p_2$ (presuming a $\pi$-shifting phase grating G1). For more information (incorporated herein by reference) see Tilman Donath et al, "Inverse geometry for grating based x-ray phase contrast imaging", JOURNAL OF APPLIED PHYSICS 106, 054703, 2009."

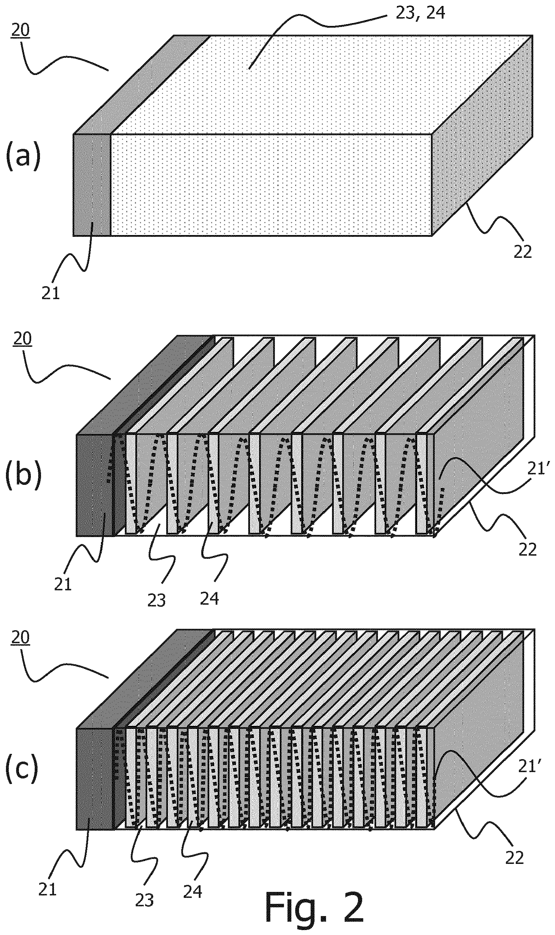

Fig. 2a schematically depicts a switchable grating 20 comprising a reservoir 22, filled with a suspension of (preferably micrometer or nanometer sized) x-ray absorbing particles 24 in a medium 23, such as for instance regular water or another liquid which has negligible x-ray absorption, such as for instance oils, salt water, distilled water, acetone, alcohol and mixtures thereof. The choice of medium is part of the degrees of freedom of the system. An adequate medium may be chosen to achieve an equivalent speed of sound that allows a practical implementation. Gold particles are particularly suitable as x-ray absorbing particles 24, since they have high absorption and no solubility in most liquids, including water. Other materials, such as tungsten, molybdenum, silver or combinations of materials, polymeric materials, for instance blended or filled with metals, may be suitable as well.

The reservoir 22 is at least acoustically, connected to an ultrasound generator 21.

The reservoir 22 has a minimum thickness to allow the particles 24 to arrange to the full height to form grating structures. The height is comparable with the length of regular, non-switchable gratings used in phase contrast imaging, preferably between 100 and 300 micron, more preferably between 150 and 250 micron and most preferably about 200 micron. Preferably the standing soundwave forces the particles 24 to organize in structures substantially extending from the bottom to the top reservoir to the top. However it may be possible that there remains a non-used volume above the grating structures in the reservoir 22. The thickness of the reservoir 22 should increase or decrease if the used particles 24 absorb less or more respectively than a common grating.

The reservoir 22 should be constructed of a material that has negligible x-ray absorption. Glass is a particularly suitable material. Also most plastics would be suitable, provided that the walls are sufficiently thin. The thickness of the reservoir walls should be such that there is a balance between reducing x-ray absorption and structural integrity.

Upon exposing the suspension 23, 24 to a strong ultrasound signal 21' generated by the ultrasound generator 21, standing waves are generated causing the particles 24 to align along pressure nodes of the standing wave, as is schematically shown in Fig. 2b. By properly selecting an excitation frequency and phase, the particles 24 will from slabs or walls along the volume of the medium separated by the distance between adjacent pressure nodes (i.e. half the ultrasound wavelength). For example, the speed of sound in water is approximately 1500 m/s. If an ultrasound source of 25 MHz is used, the particles will align and form pillars at 30 μm intervals:

$$\lambda/2 = \frac{c}{2v} = \frac{1500\frac{m}{s}}{2 \cdot 25\, MHz} = 30\mu m.$$

During ultrasound excitation the walls or slabs remain in place, forming an equivalent grating with an absorbing pitch of 30 μm that is suitable for use as an analyzer grating G2. For a sufficiently absorbing cross-section, the ultrasound transducer(s) must be such that a standing wave is generated across the full volume of the suspension exposed to the x-ray beam.

The ultrasound generator 21 comprises an ultrasound transducer that is used to stablish a standing wave 21' across the water (or other) filled container. The ultrasound transducer may be a single transducer or consist of a plurality of smaller transducers each tuned to generate a standing wave.

In an example the particles 24 are gold particles. The medium 23 is water and the cross-section of the reservoir 22 is 0,2 mm. Fig. 2a the transducer is off and all particles 24 are scattered across the medium 23. In this embodiment use is made of reflections at the opposite wall of the reservoir 22 to force a standing wave. As discussed later, it will be beneficial to actively remove the particles away from the field of view when the transducer is switched off. As soon as the transducer is switched on (Fig. 2b), the particles 24 align each pressure node (30 μm) forming gold walls alternating with equally sized water layers.

An interesting additional useful property of ultrasound generated grating structures is the flexibility of their formation and modification by means of proper beam forming techniques. For example, the modification of the speed of sound of the suspension, e.g. by dilution would cause the speed of sound to change and with it the periodicity of the structures formed. The same effect can be achieved by change in the ultrasound frequency. The addition of higher harmonics with the correct phase and amplitude by means of Fourier synthesis might be used to manipulate and improve the profile of the resulting attenuating walls (steeper profile).

As an example, in Fig. 2c the ultrasound 21 is operated such that a standing wave 21' such that gold walls with a smaller pitch, e.g. 15 μm, are formed.

The embodiments known from the prior art and as described above and shown in Fig. 2 are only able to form a grating in one direction, which therefore does not allow resolving phase gradients and scattering structures in other directions.

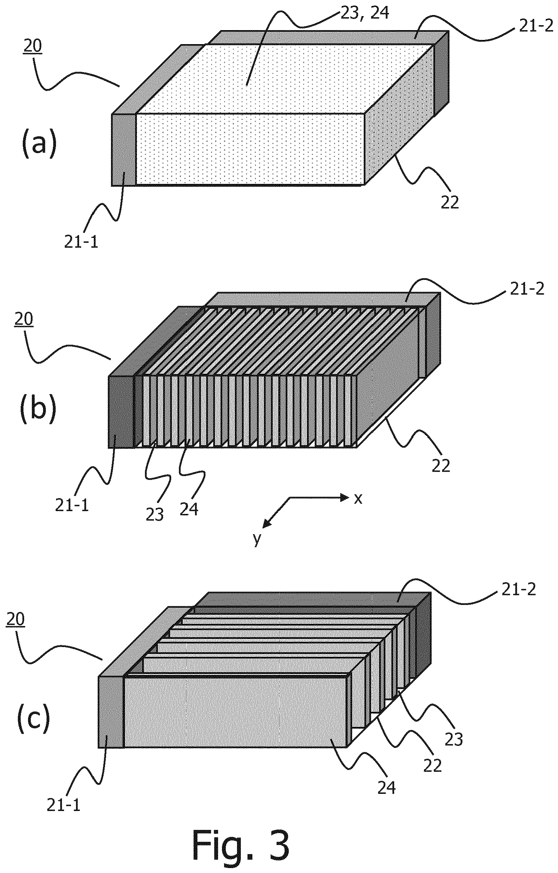

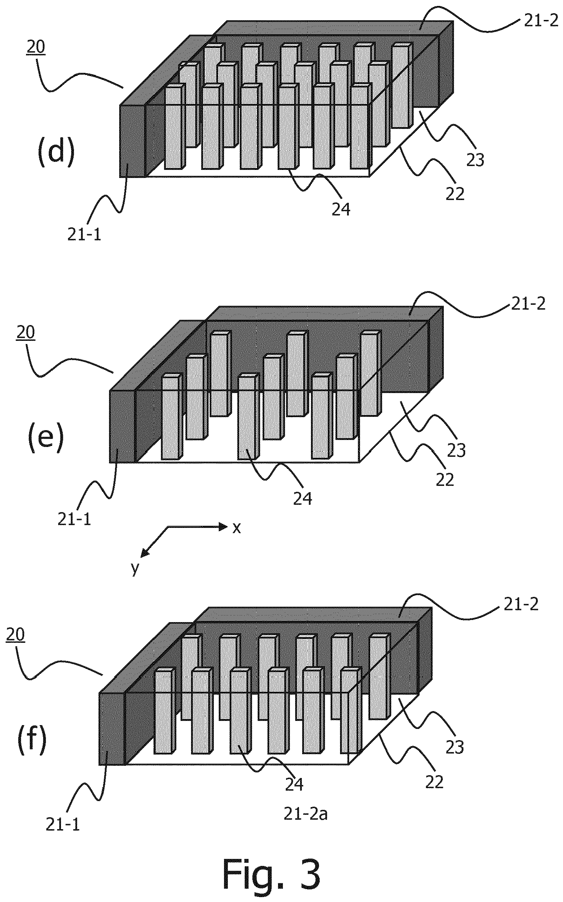

In addition to a first ultrasound generator 21-1 on a first side of the reservoir 22 as described previously, adding a second ultrasound generator 21-2 on a second side of the reservoir 22, adjacent and orthogonal to the first side, as shown in Fig. 3.

While it is known from the prior art to introduce a second ultrasound generator to the reservoir, these are only placed at the side of the reservoir 22 opposite of the first ultrasound generator 21-1. This is useful in case reflections at the opposite wall are not sufficient or possible (e.g. due to geometry or size) to force a standing wave throughout the medium 23.

The additional ultrasound generator 21-2 maybe used to generate a standing wave in the orthogonal direction compared to those generated by the first ultrasound generator 21-1. That is, the attenuating wall may be formed at a 90° angle with respect to the case discussed previously. This allows to selectively decide the direction of the analyzer grating and therefore allow resolving phase gradients and scattering structures in two distinct directions to provide even further or more detailed information of the scanned object.

Fig. 3a depicts a state in which both the first ultrasound generator 21-1 and the second ultrasound generator 21-2 are switched off. The suspension of the particles 24 in the medium 23 has a random distribution and, in case an x-ray beam is switched on and transmitted through the reservoir 22, the beam would be more or less evenly attenuated throughout the reservoir 22.

Fig. 3b depicts a state in which the first ultrasound generator 21-1 is switched on and the second ultrasound generator 21-2 is switched off. This corresponds with the situation as described previously with respect to the embodiments as shown in Fig. 2. The standing wave is formed in a first direction x away from the ultrasound generator and the particles 24 form grating walls parallel to the first side of the reservoir 22.

Fig. 3c depicts a state in which the first ultrasound generator 21-1 is switched off and the second ultrasound generator 21-2 is switched on. The standing waves are now formed in a second direction y away from the second ultrasound generator 21-2 and the particles 24 form grating walls parallel to the second side of the reservoir 22. The resulting grating structure is orthogonal with respect to the grating structure that can be formed by the first ultrasound generator 21-1.

Phase contrast imaging devices employing linear gratings break the rotational symmetry of the imaging system and provide sensitivity to phase-gratings and scattering structures only in the direction perpendicular to the grating structures. This is a severe shortcoming in clinical practice as the orientation of clinically relevant anatomical structures are a priori unknown. It is thus of high importance to provide means to overcome the directional properties of such systems and allow for sensing phase and dark-field information also in a direction perpendicular to the said first direction defined by the gratings. With the presently claimed invention it is possible to resolve phase contrast data for each location in two directions perpendicular to each other, whereby the acquired phase contrast data in each direction may be used complimentary to detect anisotropies in the imaged material, grating structures and/or the x-ray phase wave. Also some of the absorption in the material may be better separated from the phase contrast signal.

An imaging procedure may comprise imaging in one direction only, whereby the chosen direction of the switchable grating 20 is chosen such that it is most suitable for the object to be imaged. Alternatively the object may be imaged with the switchable grating 20 in a first direction and then again with the switchable grating in the second position. These may be performed consecutively or alternatingly.

When the switchable grating 20 is the analyzer grating G2, then switching the grating direction must be accompanied by a 90° of the source grating G0 and phase grating G1 grating too, which could be implemented by mechanical means or by also implementing these as switchable gratings.

In Fig. 3d both the first ultrasound generator 21-1 and the second ultrasound generator 21-2 are switched on. The first ultrasound generator 21-1 generates a first standing wave in the first direction x and the second ultrasound generator 21-2 generates a second standing wave in the second direction y. The particles 24 do not form walls, but instead form pillars around pressure nodes formed by the overlapping first soundwave and the second soundwave.

Such a 2D (as seen from the top, 3D in the volume) arrangement of pillars has a spatial distribution that depends on the frequency of both ultrasound generators 21-1, 21-2. In this way one can change the pitch of the pillars on either direction. The other two gratings in the interferometer also need to be 2D to obtain this effect.

This is shown in Fig. 3e for a case wherein, compared to the embodiment shown in Fig. 3c, the wave generated by the first ultrasound generator 21-1 now has a lower frequency, thereby forming less and farther spaced pillars in the first direction x in the medium 23. In Fig.. 3f the same is shown, but now for a case wherein, compared to the embodiment shown in Fig. 3c, the wave generated by the second ultrasound generator 21-2 now has a lower frequency, thereby forming less and farther spaced pillars in the first direction y in the medium 23.

Increasing the frequency results in more, closer spaced pillars, while decreasing the frequency results in less pillars spaced further from each other. As such, by tuning the frequency of each of the ultrasound generators 21-1, 21-2, pillars may be formed with a large two-dimensional degree of freedom in position and spacing.

The advantage of using such 2D arrangement of pillars is increased sensitivity to fine scattering structures in both directions in a single imaging procedure. The other grating structures must therefore be structured in two dimensions.

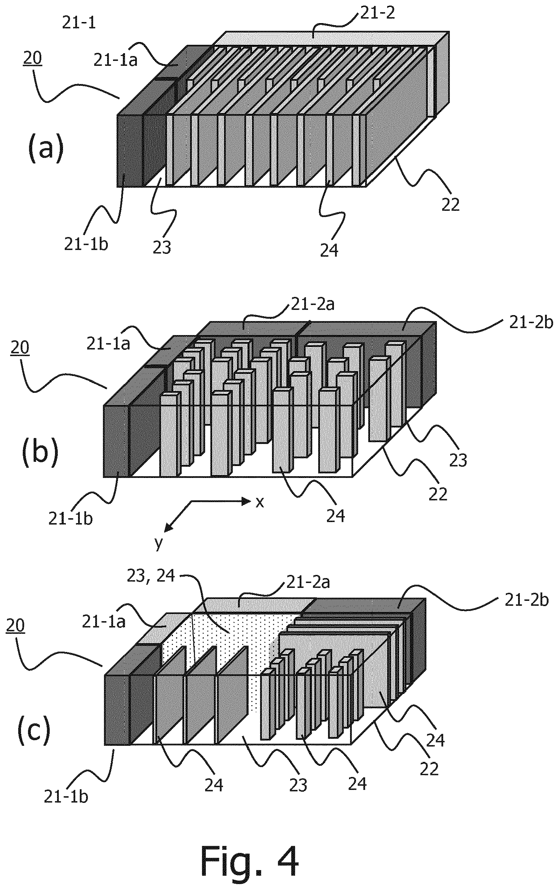

The ultrasound generator 21-1, 21-2 may comprise more than one ultrasound transducer that may be operated with independent excitation operating at the same or at a different frequency, thereby allowing for generating different wave patterns in one direction. Fig. 4 schematically illustrates several options of ultrasound generators 21-1, 21-2 with multiple transducers.

In Fig. 4a only one ultrasound generator has multiple transducers 21-2a, 21-2b with independent excitation. In this example the second ultrasound generator 21-2, but it could also be the first ultrasound generator 21-1 or both.

In this example a first transducer 21-1a of the first ultrasound generator 21-1 operates at double the frequency than that of the second transducer 21-1b, resulting in formation of standing walls that are spaced twice as much in the area extending from the second transducer 21-lb towards the opposite wall of the reservoir 22 than in the area extending from the first transducer 21-1a towards the opposite wall of the reservoir 22.

In Fig. 4b both ultrasound generators 21-1, 21-2 have two transducers 21-1a, 21-lb, 21-2a, 21-2b, that each may be operated individually at a selected frequency and phase. In this example the first transducer 21-1a of the first ultrasound generator 21-1 and the first transducer 21-2a of the second ultrasound generator 21-2 are operated at the same frequency thereby forming a regular array of pillars in the quadrant of the reservoir 22 covered by them both. The second transducer 21-1b of the first ultrasound generator 21-1 and the second transducer 21-2b of the second ultrasound generator 21-2 are operated at lower frequencies than the first transducers 21-1a, 21-2a, as well as differently from each other. This results in differently spaced pillar arrangements in each quadrant.

In Fig. 4c the first transducer 21-1a of the first ultrasound generator and the first transducer 21-2a of the second ultrasound generator are switched off and only the second transducers 21-1b, 21-2b of each ultrasound generator 21-1, 21-2 are operated. In this example at the same frequency. The result is that the particles 23 in the suspension with the medium 24 in the quadrant covered by the first transducers 21-1a, 21-2a are not pushed into walls or pillars, thereby forming a more or less homogeneously attenuating path that may be used for transmission imaging or as a reference area for the other areas if the particle density is known. The particles 24 in the quadrant covered by the second transducers 21-lb, 21-2b are driven into pillar structures by the overlapping sound waves, while the particles 24 in the remaining two quadrants are pushed into walls since they only experience one soundwave. These walls are perpendicular towards each other and may therefore provide anisotropic phase contrast information within one shot. When implemented in a computed tomography device shots from different angels complement each other and it maybe possible to generate a full image of the irradiated area of the object 50 for each of the four quadrants.

Of course many more variations are possible, such as operating the individual transducers 21-1a, 21-1b, 21-2a, 21-2b at different frequencies to influence the positions of walls, pillars and non-organized areas. They may also be operated alternatingly at different frequencies, such that for instance each area has pillars, walls or unorganized particles 24 for a certain amount of time (e.g. for a succession of several shots, for instance 4 shots in which each quadrant experiences one state of organization). Further it would of course be possible to equip each or both ultrasound generator 21-1, 21-2 with one, two, three or even more transducers for even more options. Of course this would also work for an embodiment wherein only one ultrasound generator 21-1, 21-2 is present, whereby structures such as shown in Fig. 4a may be formed (or perpendicular if only the second ultrasound generator 21-2 is present). In such a case each transducer 21-1a, 21-1b may again be operated at the same or different frequencies or phases or may be shut off or alternated between different states.

It may be beneficial to equip the switchable grating 20 with an additional ultrasound generator on the opposite side of the reservoir facing the first ultrasound generator 21-1 and/or second ultrasound generator 21-2. This is particularly advantageous if dimensions of the reservoir 22 are too large or reflection of the opposite walls are insufficient to fully form a standing wave across the entire length and/or width of the reservoir 22. The opposite generators then must be operated at the same frequency as the first and/or second ultrasound generators 21-1, 21-2.

When more than one transducer is used, regardless whether they are on the same side of the reservoir or not, then the driving voltages on the transducers involved should be phase locked. Such a phase locking mechanism is necessary to correct for jitter and drifts. However, since the position of the walls, slabs or pillars is, in first degree, only a function of the geometry and the frequency, one cannot avoid stringent requirements on the accuracy of the generators. When there are transducers on both opposite sides of the reservoir, then it is a mandatory condition that they are phase locked to ensure positional accuracy.

Imagers equipped with a phase contrast grating arrangement may be used for regular transmission imaging as well. Actually, a transmission image is obtained simultaneously with the differential phase contrast and dark field signal, but the signal is incomplete due to attenuation of the gratings G0, G1, G2. For instance, when an analyzer grating G2 is present, then about 50% of the radiation reaching the analyzer grating G2 is attenuated just before it reaches the detector 14. This is particularly problematic when the imager is used for medical transmission x-ray imaging, since 50% of the dose that already passed the object is not used for the actual imaging, thereby unnecessarily exposing the object to too much harmful ionizing radiation.

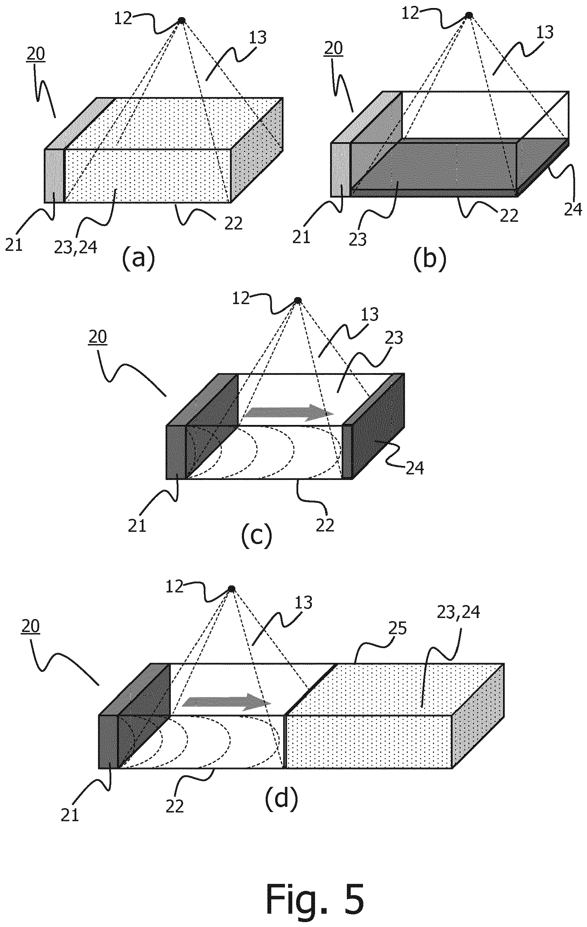

To avoid this, the grating(s) G0, G1, G2 must be removed from the system, but this will cause extensive repositioning and calibration when the grating arrangement is used again for a later imaging procedure. With a known switchable grating, in the off-state the particles 24 are in suspension (as shown in Fig. 5a) or they may have precipitated to the bottom of the reservoir (as is shown in Fig. 5b). Since the x-ray absorbing particles still remain in the path of the x-ray beam 13, even in the off-state, they have a non-negligible x-ray absorption for radiation that already passed the object and to avoid this the known switchable grating must still be removed from the path of the radiation beam 13.

To obtain a true transmission image without attenuation by the x-ray absorbing particles 24 in the reservoir 22, they should be removed from the path of the x-ray beam when the transducer is switched off. It is therefore preferable to ensure that they are displaced out of the field-of-view of the x-ray beam 13. The x-ray field-of-view is depicted in Fig. 5 as the intersection of the beam 13 emanating from the x-ray source 12 with the reservoir 22. Dimensions are highly exaggerated in these images, the actual reservoir has a very small thickness compared to the beam path length and substantially the full top area of the reservoir 22 will be irradiated by the x-ray beam 13 and the x-ray field-of-view is therefore, in this example, nearly identical to the reservoir 22 top and bottom areas.

Fig. 5c depicts an embodiment of how to remove the particles 24 from the x-ray field-of-view. In these embodiments the particles 24 are moved to a side of the reservoir 22 that is outside the x-ray field-of-view. In one example the ultrasound generator 21 maybe used to produce a standing soundwave with a frequency such that the particles are moved to the opposite side or bursts of standing waves or a travelling soundwave may be generated that sweeps the particles along to the opposite side. The ultrasound generator 21 needs to remain switched on during the imaging procedure in this case, else the particles are likely to migrate back into the x-ray field-of-view 13.

Alternatively, a membrane or plate moving from one side of the reservoir 22 to the other may sweep up all particles and transport them to the side of the reservoir. This has the advantage that the ultrasound device does not need to be used in the off-state of the switchable grating, but it would make the construction of the switchable grating somewhat more complex.

Preferably the reservoir 22 volume is slightly bigger than the x-ray field-of-view intersecting with the reservoir 22 such that there is some space to move the particles 24 fully outside the x-ray field-of-view. Alternatively there may be a second reservoir 25 connected to first reservoir which is arranged to receive the particles 24 that are moved to the side.

The second reservoir 25 must be sufficiently large to receive all the particles 24, but in an embodiment it is large enough to also receive the medium 23 from the first reservoir 22, as is shown in Fig. 5d. Although a reservoir with, for instance, a 200 $\mu$m cross-section with the medium 23 being water may be negligible in terms of x-ray absorption and would be suitable for use in the presently claimed invention, removing the medium 23 as well might increase the transmission signal even more.

In case the ultrasound generator 21 is used to sweep the particles 24 (with or without the medium 23) to the second reservoir 25, then either the ultrasound generator 21 needs to remain switched on or there must be a valve, lock, gate or other temporary wall-like construction preventing flow of the particles 24 or of particles 24 and the medium 23 to the first reservoir 22 until it is necessary to switch the switchable grating 20 on for a phase contrast imaging procedure.

The second reservoir 25 may be directly connected to the first reservoir 22 or it may be placed away from the first reservoir 22 connected by a conduit such as a pipe or hose.

In other embodiments the medium 23 and/or the particles 24 may also be flushed (using, for instance, gravity) or pumped to and from the second reservoir 25.

The presently claimed invention allows for useful application in a clinical environment such as a hospital. More specifically, the present invention is very suitable for application in imaging modalities such as mammography, diagnostic radiology, interventional radiology and computed tomography (CT) for the medical examination of patients.

In addition, the presently claimed invention allows for useful application in an industrial environment. More specifically, the present invention is very suitable for application in non-destructive testing (e.g. analysis as to composition, structure and/or qualities of biological as well non-biological samples) as well as security scanning (e.g. scanning of luggage on airports).

Further, the presently claimed invention may be useful outside of actual imaging applications. For instance, when the medium comprises a curable material, e.g. a UV or thermally curing resin, it is possible to produce highly precise (permanent) grating structures that may be used as, for instance, non-switchable gratings for phase contrast imaging or any other application which utilizes gratings. This may be achieved by acoustically coupling one or more ultrasound generators to a reservoir filled with, for instance, a low x-ray absorbing epoxy in which gold particles are suspended. After the ultrasound generator or the ultrasound generators are switched on the particles organize themselves in wall-like or pillar-like structures, after which the epoxy is cured, e.g. thermally. The reservoir may then be decoupled (acoustically and mechanically) from the reservoir. The reservoir now comprises a highly precise fixed regular x-ray absorbing structure.

Instead of changing the frequency, the pitch and position of the walls, slabs and/or pillars may also be influenced by exploiting reflections or using an opposite transducer. Alternatively the speed of sound in the medium 23 may be changed, e.g. by changing the temperature or pressure of the medium 23.

The terms 'object' or 'subject' should each in light of the present invention be understood as an inanimate object, e.g. a material for structural or other testing or objects for security checks, or a human or animal subject for, for instance, a medical diagnosis imaging scan.

The invention is suitable for any kind of x-ray phase contrast imaging, such as 2D x-ray imaging, x-ray tomosynthesis or computed tomography.

The terms 'first', 'second', 'further', etc. indicate options and are not limited to a particular sequence or order unless specified. The term 'second' may occur without the presence of a 'first'.

The term substantially means at least >50%, preferably >75%, more preferably >85%, even more prefereably>90% and most preferably>95%.

The term 'more or less' means virtually exact, accounting for naturally occurring, practical or statistical distributions and specifically it means at least >90%, preferably >95%, more preferably>99% and most preferably exactly 100%.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality.

A single unit may fulfill the functions of several items recited in the claims.

The mere fact that certain measures are recited in mutually different dependent claims does not indicate that a combination of these measured cannot be used to advantage.

Any reference signs in the claims should not be construed as limiting the scope.

## Claims

**1.** A switchable grating (20) for a differential phase contrast imaging and/or dark-field imaging interferometer comprising:

- a reservoir (22) containing a substantially x-ray transparent medium (23) comprising particles (24) that substantially attenuate x-ray radiation;
- a first ultrasound generator (21-1) arranged along a first side of the reservoir configured to generate a first soundwave with a frequency and phase such that a first standing wave is formed within the medium causing the x-ray absorbing particles to organize along pressure nodes of the first standing wave;
- a second ultrasound generator (21-2) arranged along a second side of the reservoir orthogonal to the first side configured to generate a second soundwave with a frequency and phase such that a second standing wave is formed within the medium causing the x-ray absorbing particles to organize along pressure nodes of the second standing wave.

2. Switchable grating (20) according to claim 1 wherein the first ultrasound generator (21-1) and second ultrasound generator (21-2) are configured to operate separately, alternatingly and/or simultaneously.

3. Switchable grating (20) according to claim 1 or 2, wherein the first ultrasound generator (21-1) and second ultrasound generator (21-2) are configured to independently generate a soundwave at a selected frequency.

4. Switchable grating (20) according to any of the previous claims, wherein the first ultrasound generator (21-1) and/or the second ultrasound generator (21-2) comprise at least two ultrasound transducers (21-1a, 21-1b, 21-2a, 21-2b) spaced next to each other, each of said transducers configured to independently generate a soundwave at a selected frequency.

5. Switchable grating (20) according to any of the previous claims, further comprising:

- a third ultrasound generator arranged along a third side of the reservoir opposite to the first side configured to generate the first soundwave with a frequency and phase such that the first standing wave is formed within the medium causing the particles to organize along pressure nodes of the first standing wave; and/or
- a fourth ultrasound generator arranged along a fourth side of the reservoir opposite to the second side configured to generate the second soundwave with a frequency and phase such that the second standing wave is formed within the medium causing the particles to organize along pressure nodes of the second standing wave.

6. A switchable grating (20) for a differential phase contrast imaging and/or dark-field imaging interferometer comprising:

- a reservoir (22) containing a substantially x-ray transparent medium (23) comprising particles (24) that substantially attenuate x-ray radiation;
- a first ultrasound generator (21-1) arranged along a first side of the reservoir configured to generate a first soundwave;
- means for flushing the reservoir to remove the particles, and optionally the medium, outside a field-of-view of an x-ray beam (13) as used in an imaging procedure.

7. Switchable grating (20) according to claim 6, wherein the means for flushing the reservoir (22) is arranged to flush the particles (24), and optionally the medium (23), to a side of the reservoir or to a container (25) removed from the reservoir.

8. Switchable grating (20) according to claim 6 or 7, wherein the means for flushing the reservoir is configured to flush the particles (24), and optionally the medium (23), by generating a propagating soundwave from the first ultrasound generator (21-1) such that the propagating soundwave carries substantially all particles outside a field-of-view of an x-ray beam (13) during an imaging procedure.

9. A system for differential phase contrast imaging and/or dark-field imaging comprising:

- a coherent polychromatic or an incoherent polychromatic x-ray source (11) provided with a source grating (G0) in transmission geometry for making the x-ray beam coherent;
- an interferometer comprising at least a phase grating (G1) in transmission geometry for generating a pattern of interference fringes at a certain location downstream the optical axis;
- an x-ray detector (14) having sufficient spatial resolution for resolving a fringe pattern generated by phase grating (G1) or a conventional X-ray detector provided with an analyzer grating (G2) in transmission geometry for resolving the interference fringes generated by the phase grating (G1),
- wherein the interferometer further comprises, when present, the source grating (G0), and/or the analyzer grating (G2), and the interferometer is installed between the x-ray source and x-ray detector; **characterized in that**
- at least one of the phase grating (G1) or, when present, the source grating (G0) or, when present, analyzer grating (G2) is a switchable grating (20) according to any of the previous claims.

10. System according to claim 9, wherein the analyzer grating (G2) is present and is the switchable grating (20).

11. System according to claim 9 or 10, wherein the phase grating (G1) and, when present, the source grating (G0) are rotatable by 90 degrees or are switchable gratings (20) according to any of the claims 1 to 5.

12. A method for differential phase contrast imaging and/or dark-field imaging of an object comprising the steps of:

    - placing the object in an examination area of a system for differential phase contrast imaging and/or dark-field imaging according to any of the claims 10-11;
    - generating a first soundwave in a first direction with a frequency and phase such that a first standing wave is formed within the medium causing the particles to organize along pressure nodes of the first standing wave, thereby forming the analyzer grating (G2); and/or
    - generating a second soundwave in a second direction, orthogonal to the first direction, with a frequency and phase such that a second standing wave is formed within the medium causing the particles to organize along pressure nodes of the first standing wave, thereby forming the analyzer grating (G2).

13. Method according to claim 12, wherein the first soundwave is switched off followed by generation of the second soundwave, preferably after the phase grating (G1) and, when present, the source grating (G0) are rotated by 90 degrees from a first position to a second position or are switchable gratings according to any of the claims 1 to 5 that are switched between a sound wave in the first direction to a second soundwave in the second direction, corresponding to the respective first direction and second direction of the switchable analyzer grating (G2).

14. Method according to claim 12, wherein the first soundwave and the second soundwave are generated simultaneously.

15. Method to manufacture a grating for differential phase contrast imaging and/or dark-field imaging comprising the of the method according to any of the claims 12 to 14, wherein the particles are curable particles that are cured after the particles have organized themselves along the pressure nodes of the standing wave(s) in a curing step, preferably a thermal curing step or a radiation curing step.

10

12

11

G0

13

G1

50

G2

14

# Fig. 1

Fig. 2

Fig. 3

Fig. 3

Fig. 4

Fig. 5

Europäisches
Patentamt

European
Patent Office

Office européen
des brevets

# EUROPEAN SEARCH REPORT

Application Number

EP 18 19 5665

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | US 2007/183584 A1 (BAUMANN JOACHIM [DE] ET AL) 9 August 2007 (2007-08-09) | 6,7 | INV. A61B6/00 |
| Y | * figures 1,5 * | 1-5,9-14 | G01N23/20 |
| A | | 8 | G02F1/11 G02F1/29 |
| X | US 2007/189449 A1 (BAUMANN JOACHIM [DE] ET AL) 16 August 2007 (2007-08-16) | 6,7 | G02F1/33 G02B5/18 |
| Y | * paragraph [0040]; figure 1 * | 1-4,9-14 | G02B27/50 |
| A | | 8 | G21K1/06 G01N23/041 |
| Y | DE 955 476 C (KOCH & STERZEL AG) 3 January 1957 (1957-01-03) | 1-5,9-14 | G02F1/165 G21K1/04 |
| A | * col. 2, line 61-63 * | 8 | G03F7/20 |
| A | Gerhard Kütterer: "Lexikon der röntgenologischen Technik 1895 bis 1925 von Abdeckzunge bis Zylinderblende" In: "Lexikon der röntgenologischen Technik 1895 bis 1925 von Abdeckzunge bis Zylinderblende", 1 January 2017 (2017-01-01), BoD - Books on Demand, XP055566274, ISBN: 978-3-7448-5013-1 page 27, * the whole document * | 8 | ADD. G01V5/00 G02B21/14 G02B27/52 |
| X | JP H08 190008 A (NAKAGAWA YASUHIKO) 23 July 1996 (1996-07-23) * paragraph [0039]; figure 8 * | 15 | TECHNICAL FIELDS SEARCHED (IPC) A61B H05G G01V G01N G02F G02B G21K G03F |

The present search report has been drawn up for all claims

| Place of search | Date of completion of the search | Examiner |
|---|---|---|
| Munich | 8 March 2019 | Anscombe, Marcel |

EPO FORM 1503 03.82 (P04C01)

## ANNEX TO THE EUROPEAN SEARCH REPORT
## ON EUROPEAN PATENT APPLICATION NO.

EP 18 19 5665

This annex lists the patent family members relating to the patent documents cited in the above-mentioned European search report.
The members are as contained in the European Patent Office EDP file on
The European Patent Office is in no way liable for these particulars which are merely given for the purpose of information.

08-03-2019

| Patent document cited in search report | Publication date | Patent family member(s) | Publication date |
|---|---|---|---|
| US 2007183584 A1 | 09-08-2007 | DE 102006037282 A1<br>JP 2007203060 A<br>US 2007183584 A1 | 09-08-2007<br>16-08-2007<br>09-08-2007 |
| US 2007189449 A1 | 16-08-2007 | DE 102006037257 A1<br>JP 2007206076 A<br>US 2007189449 A1 | 02-08-2007<br>16-08-2007<br>16-08-2007 |
| DE 955476 C | 03-01-1957 | NONE | |
| JP H08190008 A | 23-07-1996 | NONE | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20070183584 A1

- US 20140177795 A1

**Non-patent literature cited in the description**

- **PFEIFFER.** Hard-X-ray dark-field imaging using a grating interferometer. *Nature Materials,* 07 February 2008, 134-137

- **TILMAN DONATH et al.** Inverse geometry for grating based x-ray phase contrast imaging. *JOURNAL OF APPLIED PHYSICS,* 2009, vol. 106, 054703